# EUROPÄISCHE PATENTSCHRIFT

(11) **EP 0 257 416 B1**
(45) Veröffentlichungstag und Bekanntmachung des Hinweises auf die Patenterteilung: **29.09.1993**
(21) Anmeldenummer: 87111508.5
(22) Anmeldetag: 08.08.1987
(51) Int. Cl.: C12N 15/65

(54) **Farbmarker für Klonierungen in Streptomyces lividans**
Colour marker for cloning in streptomyces lividans
Marqueur de couleur pour le clonage dans les Stréptomyces lividans

(30) Priorität: 13.08.1986 DE 3627392
(43) Veröffentlichungstag der Anmeldung: 02.03.1988
(73) Patentinhaber: HOECHST AKTIENGESELLSCHAFT, 65926 Frankfurt am Main (DE)
(72) Erfinder: Muth, Günter, D-4800 Bielefeld (DE); Wohlleben, Wolfgang, Dr., D-4800 Bielefeld (DE); Pühler, Alfred, Prof. Dr., D-4800 Bielefeld (DE); Wöhner, Gerhard, Dr., D-6093 Flörsheim am Main (DE); Marquardt, Rüdiger, Dr., D-6000 Frankfurt am Main (DE)

(56) Entgegenhaltungen:
- EP-A- 0 154 430
- CHEMICAL ABSTRACTS, Band 98, Nr. 7, Februar 1983, Seite 180, Zusammenfassung Nr. 47927w, Columbus, Ohio, US; E.S. PIRUZYAN et al.: "Cloning of DNA fragments of Streptomyces coelicolor A3(2) plasmid SCP2 in Escherichia coli cells", & GENETIKA (MOSCOW) 1982, 18(11), 1811-16
- JOURNAL OF BACTERIOLOGY, Band 162, Nr. 1, April 1985, Seiten 406-412, American Society for Microbiology; S. HORINOUCHI et al.: "Construction and appplication of a promoter-probe plasmid that allows chromogenic identification in Streptomyces lividans"
- CURR. TOP. MICROBIOL. IMMUNOL., Band 96, 1982, Seiten 69-95; K.F. CHATER et al.: "Gene cloning in Streptomyces"
- JOURNAL OF GENERAL MICROBIOLOGY, Band 131, 1985, Seiten 2431-2441, SGM: J.S. FEITELSON et al.: "Genetic and biochemical characterization of the red gene cluster of Streptomyces coelicolor A3(2)"
- JOURNAL OF NATURAL PRODUCTS, Band 49, Nr. 6, November/Dezember 1986, Seiten 988-994; J.S. FEITELSON et al.: "Molecular genetics of red biosynthesis in Streptomyces"

## Beschreibung

Als Farbmarker für die Klonierung in Streptomyceten ist bisher das mel-Gen bekannt (E. Katz et al., J. Gen. Microbiol. 129 (1983) 2703), das für Tyrosinase codiert und damit - über Zwischenstufen - für die Produktion des Farbstoffes Melanin verantwortlich ist. Dieses Gen ist beispielsweise in dem handelsüblichen Plasmid pIJ702 enthalten, das von der John Innes Foundation, Norwich, England, erhältlich ist und beispielsweise in D. A. Hopwood et al., Genetic Manipulation of Streptomyces - A Laboratory Manual, The John Innes Foundation, 1985, S. 292 f., beschrieben ist.

Ein weiteres braunes Pigmentgen ist aus S. Horinouchi und T. Beppu (1985), J. Bacteriol, 162, 406-412, No. 1 bekannt.

Es wurden nun DNA-Fragmente gefunden, die in Streptomyces lividans exprimiert werden und im Medium eine purpurrote intensive Färbung bewirken, die in festen Medien eine geringe Diffusion zeigt.

Die Erfindung betrifft somit DNA-Fragmente, die die Expression eines roten Farbstoffs in Streptomyces lividans bewirken. Diese sind erhältlich aus der Gesamt-DNA von Streptomyces coelicolor DSM 3030 durch Schneiden mit BamHI, Klonieren der Fragmente in einen geeigneten Vektor und Selektion auf rote Farbstoffproduktion. Der Ausgangsstamm S. coelicolor DSM 3030 ist in der Europäischen Patentanmeldung mit der Veröffentlichungsnummer 0 181 562 als Produzent eines bakterienlysierenden Enzyms genannt.

G. Habermehl et al., Z. Naturforsch. 32b (1977) 1195, beschreiben die Isolierung, Auftrennung und Strukturaufklärung des blauen Bakterienpigments "Amylocyanin" aus Streptomyces coelicolor Müller, DSM 40665. Neben diesem wasserlöslichen blauen Farbstoff werden in der späten Wachstumsphase noch weitere, nicht näher identifizierte Pigmente produziert, darunter auch rote Substanzen.

Die Erfindung bezieht sich weiterhin auf die Verwendung der erfindungsgemäßen DNA-Fragmente als Marker, insbesondere als Inaktivierungs-Marker, in Streptomyceten-Plasmiden.

Weitere Aspekte der Erfindung und ihre bevorzugten Ausgestaltungen ergeben sich aus der folgenden Beschreibung bzw. aus den Patentansprüchen.

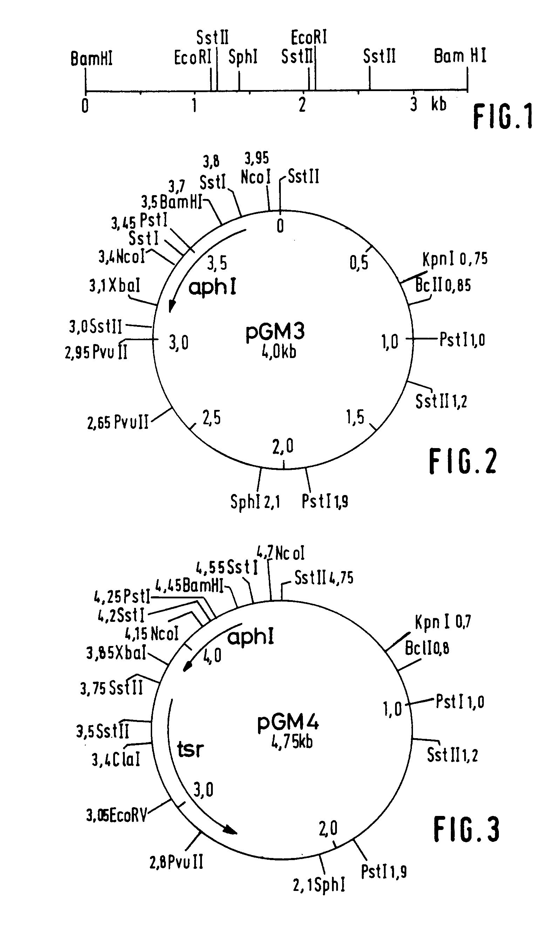

Zur Isolierung der DNA-Fragmente isoliert man die Gesamt-DNA aus dem Stamm Streptomyces coelicolor DSM 3030 durch Schneiden mit dem Restriktionsenzym BamHI und "shot gun"-Klonierung in einen geeigneten Vektor, Transformation eines Streptomyceten-Empfängerstamms und Selektion auf Farbstoffproduktion. Die positiven Klone enthalten unterschiedliche DNA-Fragmente aus DSM 3030 von etwa 3,4 bis 9 kb. Figur 1 zeigt eine Restriktionskarte des 3,4 kb Fragmentes.

Der gebildete rote Farbstoff ist gut wasserlöslich. Die purpurrote Koloniemorphologie kommt durch eine hohe Farbstoffkonzentration zustande. In einem Festmedium bleibt der überwiegende Teil des Farbstoffs am Mycel gebunden, ein Teil diffundiert jedoch in das Medium. Unter dem Mikroskop zeigt sich ein charakteristisches Bild: Der Farbstoff leigt in dicht gedrängten kugelförmigen Körpern am Mycel und läßt somit die Kolonie intensiv purpurrot erscheinen. In Flüssigkultur (Tryptic soya broth, "Lysemedium A", Europäische Patentanmeldung mit der Veröffentlichungsnummer 0 158 872, S. 6) erhält man nach etwa 3 Tagen einen intensiv rot gefärbten Kulturüberstand. Auch in festen Medien erfolgt die Farbstoffproduktion unabhängig vom eingesetzten Medium ("R2YE" (Hopwood et al., a.a.O.), Sporulationsmedium (Deutsche Offenlegungsschrift 3 331 860, Beispiel 1, drittes Medium), "Penassay", "Penassay" mit Antibiotica-Zusatz).

Der rote Farbstoff wird bereits zu Beginn des Koloniewachstums gebildet und ist somit offensichtlich kein Produkt des sekundären Stoffwechsels. Im Gegensatz dazu produziert der Wildtyp von S. lividans erst gegen Ende des vegetativen Wachstums ein rotes Pigment (Horinouchi et al., Agric. Biol. Chem. 48 (1984) 2131).

Das 3,4 kb-Fragment gemäß Figur 1 zeigt eine Reihe von Schnittstellen, die für Subklonierungsexperimente geeignet sind. Es enthält keine Schnittstellen für die Enzyme XhoI, HindIII und PstI.

Der rote Phänotyp von plasmidhaltigen Wirtszellen ist plasmidkodiert, wie durch Plasmidisolierung und Retransformation gezeigt werden kann.

Die erfindungsgemäßen DNA-Fragmente kodieren entweder direkt für die Produktion des roten Farbstoffs oder für ein Regulationsgen, das in S. lividans diese Produktion induziert. Daß in S. prasinus und S. ghanaensis keine derartige Farbstoffproduktion erfolgt, spricht für das Vorliegen eines Regulationsgens. Damit eignen sich die erfindungsgemäßen DNA-Fragmente nicht nur generell für Klonierungsexperimente, sondern speziell zum Aufspüren von Stoffwechselwegen in Streptomyceten. Hierfür sprechen die Ergebnisse von Horinouchi et al., a.a.O. und J. Bacteriol. 158 (1984), 481-487, die gezeigt haben, daß aus S. bikiniensis ein Regulatorgen kloniert werden kann, das in S. lividans die Produktion roter Pigmente stimuliert.

In den folgenden Beispielen wird die Erfindung näher erläutert. Prozentangaben und Teile beziehen sich hierbei auf das Gewicht, sofern keine anderen Angaben gemacht sind.

Die Figuren - mit Ausnahme der Polylinkerregionen - sind maßstabsgerecht.

### Beispiel 1: Herstellung des Vektors pGM4

Aus dem Plasmid pGM1 (Europäische Patentanmeldung mit der Veröffentlichungsnummer 0 158 872, Figur 2) wird durch partielle Verdauung mit SstII ein 3,0 kb Fragment gewonnen. Aus dem Plasmid pSLE16 (Europäische Patentanmeldung mit der Veröffentlichungsnummer 0 158 201, Figur 18) wird durch Schneiden mit SstII das 1 kb-Fragment gewonnen, das das Neomycin-Resistenzgen aphI enthält. Durch Ligieren der beiden Fragmente erhält man das Plasmid pGM3 (Figur 2).

Aus pSLE41 (EP-A2 0 158 201, Fig. 20) wird außerdem durch Schneiden mit BclI ein 1 kb-Fragment isoliert, dessen überstehende Enden mit Klenow-Polymerase aufgefüllt werden. pGM3 wird nun mit PvuII geschnitten und das 0,3 kb-Fragment entfernt. Das Restplasmid wird nun mit dem stumpfendig gemachten BclI-Fragment ligiert, wobei das Plasmid pGM4 erhalten wird (Figur 3).

### Beispiel 2: Herstellung einer Genbank

S. coelicolor DSM 3030 wird lysiert und die DNA in bekannter Weise isoliert. Diese wird mit BamHI total verdaut. Das Plasmid pGM4 (Figur 3) wird mit BamHI geschnitten, mit Alkalischer Phosphatase behandelt und mit den BamHI-Fragmenten ligiert. Die so erhaltene Plasmid-Population wird in den Empfängerstamm S. lividans TK 23 (erhältlich bei John Innes Foundation) transformiert. Man erhält beim Einsatz von 1 µg Ligationsgemisch etwa 20000 Thiostrepton-resistente Transformanten, von denen 80 % Neomycin-sensitiv sind und folglich ein Insert enthalten.

### Beispiel 3: Isolierung der erfindungsgemäßen DNA-Fragmente

Unter 500 Transformanten wurde im Mittel eine intensiv purpurrot gefärbte Kolonie gefunden. Diese wurden abgetrennt und die Plasmid-DNA aus diesen Klonen isoliert. Eine nachfolgende Retransformation nach S. lividans TK 23 erbrachte nur rot gefärbte Kolonien.

Eine Charakterisierung des Plasmid-DNA ergab, daß 3,4 bis 9 kb BamHI-Fragmente in das aphI-Gen von pGM4 insertiert waren. Das Plasmid mit der 3,4 kb Insertion erhielt die Bezeichnung pGM97.

### Beispiel 4: Transformation

Das Plasmid pGM97 wurde in verschiedene handelsübliche Stämme von S. lividans transformiert. Alle zeigten eine Expression des roten Farbstoffs, sowohl in Flüssig- als auch in Festmedien.

## Patentansprüche (Patentansprüche für folgende(n) Vertragsstaat(en): BE, CH, DE, FR, GB, GR, IT, LI, LU, NL, SE)

1. DNA Fragment, erhältlich aus der Gesamt-DNA von Streptomyces coelicolor DSM 3030, gekennzeichnet durch die Restriktionskarte nach Figur 1, auf dem ein Gen lokalisiert ist, das die Expression eines roten Farbstoffs in Streptomyces lividans bewirkt.

2. Ein Plasmid, das in einem Streptomyces-Stamm replizieren kann und als Marker ein Gen enthält, das die Expression eines roten Farbstoffs in Streptomyces lividans bewirkt und das auf einem DNA-Fragment gemäß Anspruch 1 lokalisiert ist.

3. Ein Plasmid nach Anspruch 2, bei dem der Marker als Inaktivierungsmarker eingesetzt wird.

4. Streptomyces lividans-Stamm, der ein Plasmid nach Anspruch 2 oder 3 enthält.

5. Verfahren zur Isolierung eines DNA Fragmentes nach Anspruch 1, das die folgenden Schritte umfaßt
(a) Schneiden der Gesamt-DNA von Streptomyces coelicolor DSM 3030 mit BamHI,
(b) Klonieren der Fragmente in einen geeigneten Vektor,
(c) Transformation von S. lividans und
(d) Selektion auf die Bildung eines roten Farbstoffs.

## Patentansprüche (Patentansprüche für folgende(n) Vertragsstaat(en): AT, ES)

1. Verfahren zur Isolierung eines DNA Fragmentes, erhältlich aus der Gesamt-DNA von Streptomyces coelicolor DSM 3030, gekennzeichnet durch die Restriktionskarte nach Figur 1, auf dem ein Gen lokalisiert ist, das die Expression eines roten Farbstoffs in Streptomyces lividans bewirkt, das die folgenden Schritte umfaßt
(a) Schneiden der Gesamt-DNA von Streptomyces coelicolor DSM 3030 mit BamHI,
(b) Klonieren der Fragmente in einen geeigneten Vektor,
(c) Transformation von S. lividans und
(d) Selektion auf die Bildung eines roten Farbstoffs.

2. Verwendung der nach Anspruch 1 erhältlichen DNA als Marker in Streptomyceten-Plasmiden.

3. Verwendung der nach Anspruch 1 erhältlichen DNA als Inaktivierungs-Marker in Streptomyceten-Plasmiden.

## Claims (Claims for the following Contracting State(s): BE, CH, DE, FR, GB, GR, IT, LI, LU, NL, SE)

1. A DNA fragment obtainable from the total DNA of Streptomyces coelicolor DSM 3030 which has the restriction map shown in Figure 1, and on which a gene is localized which brings about the expression of a red coloring agent in Streptomyces lividans.

2. A plasmid which is able to replicate in a Streptomyces strain and contains as marker a gene which brings about the expression of a red coloring agent in Streptomyces and which is localized on a DNA fragment as claimed in claim 1.

3. A plasmid as claimed in claim 2, in which the marker is employed as inactivation marker.

4. Streptomyces lividans strain which contains a plasmid as claimed in claim 2 or 3.

5. A process for isolating a DNA fragment as claimed in claim 1, which comprises the following steps
(a) cutting the total DNA of Streptomyces coelicolor DSM 3030 with BamHI,
(b) cloning the fragments into a suitable vector,
(c) transformation of S. lividans and
(d) selection for the production of a red coloring agent.

## Claims (Claims for the following Contracting State(s): AT, ES)

1. A process for isolating a DNA fragment obtainable from the total DNA of Streptomyces coelicolor DSM 3030 which has the restriction map shown in Figure 1, and on which a gene is localized which brings about the expression of a red coloring agent in Streptomyces lividans, which comprises the following steps
(a) cutting the total DNA of Streptomyces coelicolor DSM 3030 with BamHI,
(b) cloning the fragments into a suitable vector,
(c) transformation of S. lividans and
(d) selection for the production of a red coloring agent.

2. Use of the DNA obtainable according to claim 1 as marker in Streptomyces plasmids.

3. Use of the DNA obtainable according to claim 1 as inactivation marker in Streptomyces plasmids.

## Revendications (Revendications pour l'(les) Etat(s) contractant(s) suivant(s): BE, CH, DE, FR, GB, GR, IT, LI, LU, NL, SE)

1. Fragment d'ADN, qu'on peut obtenir à partir de l'ADN total de Streptomyces coelicolor DSM 3030, caractérisé par la carte de restriction de la figure 1, sur lequel est localisé un gène qui provoque l'expression d'un colorant rouge dans Streptomyces lividans.

2. Plasmide capable de se répliquer dans une souche de Streptomyces et contenant, comme marqueur, un gène qui provoque l'expression d'un colorant rouge dans Streptomyces lividans et qui est localisé sur un fragment d'ADN conforme à la revendication 1.

3. Plasmide selon la revendication 2, dans lequel on introduit le marqueur comme marqueur d'inactivation.

4. Souche de Streptomyces lividans, qui contient un plasmide selon la revendication 2 ou 3.

5. Procédé pour isoler un fragment d'ADN selon la revendication 1, qui englobe les étapes suivantes :
(a) Coupure de l'ADN total de Streptomyces coelicolor DSM 3030 à l'aide de BamHI,
(b) Clonage des fragments dans un vecteur approprié,
(c) Transformation de S. lividans, et
(d) Sélection d'après la formation d'un colorant rouge.

## Revendications (Revendications pour l'(les) Etat(s) contractant(s) suivant(s): AT, ES)

1. Procédé pour isoler un fragment d'ADN, qu'on peut obtenir à partir de l'ADN total de Streptomyces coelicolor DSM 3030, caractérisé par la carte de restriction de la figure 1, sur lequel est localisé un gène qui provoque l'expression d'un colorant rouge dans Streptomyces lividans, lequel procédé englobe les étapes suivantes :
(a) Coupure de l'ADN total de Streptomyces coelicolor DSM 3030 à l'aide de BamHI,
(b) Clonage des fragments dans un vecteur approprié,
(c) Transformation de S. lividans, et
(d) Sélection d'après la formation d'un colorant rouge.

2. Utilisation de l'ADN qu'on peut obtenir conformément à la revendication 1, comme marqueur dans des plasmides de streptomycètes.

3. Utilisation de l'ADN qu'on peut obtenir conformément à la revendication 1, comme marqueur d'inactivation dans des plasmides de streptomycètes.
